# EUROPEAN PATENT SPECIFICATION

(11) **EP 0 746 251 B1**
(45) Date of publication and mention of the grant of the patent: **26.03.2003**
(21) Application number: 94917416.3
(22) Date of filing: 13.05.1994
(51) Int. Cl.: A61B 18/12

(54) **CATHETER FOR DIVERSE IN SITU TISSUE THERAPY**
KATHETER FÜR VERSCHIEDENE IN-SITU GEWEBETHERAPIEN
CATHETER DESTINE A DIVERSES THERAPIES TISSULAIRES IN SITU

(30) Priority: 14.05.1993 US 63094
(43) Date of publication of application: 11.12.1996
(73) Proprietor: Boston Scientific Corporation, Natick, Massachusetts 01760 (US)
(72) Inventor: DURGIN, Russell, F., Jr., Attelboro, MA 02073 (US); ROWLAND, Christopher, A., Marlborough, MA 02073 (US); SULLIVAN, Roy, H., Uxbridge, MA 01569 (US); VERGANO, Michael, G., Cumberland, RI 020864 (US)
(74) Representative: Warren, Anthony Robert
(86) International application number: US9405506
(87) International publication number: WO94026189

(56) References cited:
- US-A- 4 532 924
- US-A- 5 007 908

## Description

This invention generally relates to electro-coagulation of tissue in the body in combination with other forms of therapy using catheters.

It is very important to minimize the time required to stop internal bleeding in a patient. A physician has several medical instruments in his or her armamentarium for stopping such internal bleeding. In accordance with one modality that is particularly suited for bleeding into the gastrointestinal tract, a physician initially positions a flexible endoscope in the patient with its distal end proximate a hemorrhaging vessel. The physician may insert an irrigator through a working channel in the endoscope to clear the area by administering saline solution as a precursor to any attempts to stop bleeding. If the instrument being used for irrigation is like the Gold Probe™ hemostat manufactured by Boston Scientific Corporation, the present applicant, the physician may then cauterize the bleeding vessel using a distally positioned hemostat. Such instruments are constructed to be employed through a working channel of an endoscope to seal potential bleeding sites as in the gastrointestinal tract or the esophagus. Other hemostats use mono-electropolar electrodes in which one electrode is carried by a catheter to a site while the other electrode is an exterior ground plate placed in a patient. Alternatively the physician may retract the irrigating catheter and insert an elongated needle through the endoscope to inject a vaso-constrictor into the vessel to slow hemorrhaging. Then the physician could remove the elongated needle and reinsert the hemostat to finish the operation.

The above-mentioned Gold Probe™ hemostat is an example of a device that supplies a suitable current density and wave form of radiofrequency energy to perform electro-coagulation or cauterization. It utilizes a catheter with a bipolar electrode assembly located on a distal flexible tip formed of a ceramic cylinder having a hemispherical end. The ceramic tip includes a pair of spaced gold spiral electrodes applied to its cylindrical surface and domed end. RF energy applied to the electrodes produces a current through adjacent tissue that heats and cauterizes the hemorrhaging vessel which is contacted by the tip of the catheter.

Notwithstanding the fact that both hemostasis and injection needle therapy are usually done on an emergency basis, the exchange of catheters to provide different functions continues and increases the risk to the patient because the time to complete therapy is extended. Extending the time to complete the therapy also increases patient discomfort. Consequently, physicians have to weigh the time, complexity and benefits of interchanging single or dual purpose catheters to change treatment modalities against whatever disadvantage may result by working with a single modality and single catheter.

Attention is directed to US-A-5 403 311 which provides a catheter generally characterized by an axially displaceable probe that acts as one of two electrodes for performing hemostatic therapy. The other electrode is spaced proximally from and is insulated with respect to the probe. The catheter has a lumen for administering irrigating fluids to tissue at the hemorrhaging vessel. In some embodiments irrigation fluid passes between the main probe and a structure at the distal end of the catheter. In other applications the distal tip portion includes separate passages for allowing an irrigating solution to pass into the area of the hemorrhaging vessel. In still another embodiment the irrigation fluid passes directly through the probe that comprises a hollow needle that also acts as an electrode and as an injection needle for administering a vasoconstrictor or other therapeutic agent into a bleeding vessel.

This apparatus, therefore, in its various embodiments, enables a physician to irrigate tissue and to treat a hemorrhaging vessel with injection therapy or hemostatic therapy without removing the catheter apparatus from the working channel or lumen of an endoscope. However, many physicians are familiar with standard devices such as the Gold Probe hemostat. One particular embodiment of **US-A-5,403,311** that is applied to a Gold Probe hemostat discloses a probe tip with two separate electrodes and an extensible conductive probe that provides a physician with alternatives for hemostatic therapy. In accordance with one option the probe tip electrodes constitute bipolar electrodes and the extensible conductive probe is inactive electrically. In accordance with the other option the extensible probe is active and one or both of the probe tip electrodes act as a second electrode. This approach can complicate the apparatus required for connecting an RF generator source to the electrodes and related circuitry and can also increase the burden on the physician using the apparatus. Moreover, such procedures differ from those familiar to the physician as a result of use of prior art devices, so such a catheter assembly can impose other complications.

Reference is also made to **US-A-4,532,924** which is directed to a catheter assembly generally as defined in the preamble of claim 1, and which comprises:
catheter means having a catheter lumen therethrough from a proximal to a distal end thereof for extending from a location externally of a patient to tissue being treated, and
bipolar electrode means attached to the distal end of said catheter means for providing hemostatic therapy to the selected tissue, said bipolar electrode means having a bipolar electrode means lumen therethrough aligned with said catheter means lumen for enabling the transfer of fluids between said catheter means lumen and the selected tissue through said bipolar electrode means lumen.

**US-A-5,007,908** also discloses a catheter assembly of the general type defined in the preamble of claim 1.

It is an object of this invention to provide an integrated catheter assembly that enables a physician to select among in situ hemostatic and injection therapy modalities.

According to this invention, as defined in the characterising clause of claim 1, the catheter assembly is an integrated assembly which additionally enables a physician to utilise dual in situ therapy modalities to treat internal bleeding at selected tissue, which dual modalities comprise a hemostatic therapy modality and an injection therapy modality, and includes needle means including an elongated hollow injection needle having a needle lumen therethrough for providing injection therapy, said needle being electrically isolated from said bipolar electrode means, having an axially directed distal portion, and extending from a proximal end externally of the patient through said catheter means lumen and said bipolar electrode means lumen for axial displacement whereby said distal portion of said needle extends distally beyond and retracts proximally of the distal end surface of said bipolar electrode means through said bipolar electrode means.

Various embodiments of the present invention will now be described with reference to the accompanying drawings, in which:-
FIG. 1 is a view, partially in section, of an integrated catheter assembly extending between proximal and distal end portions that includes a catheter, an injection needle and a bipolar electrode assembly;
FIG. 2 is a detailed view, partially in section, of the distal end portion of the apparatus in FIG. 1 including the bipolar electrode assembly in which the injected needle is retracted;
FIG. 3 is a detailed view, partially in section, of the distal end portion of the apparatus in FIG. 1 in which the injection needle is extended;
FIG. 4 depicts an alternate tip structure that can be substituted for the bipolar electrode assembly shown in FIGS. 2 and 3;
FIG. 5 is a detailed plan view of an injection needle used in the structure shown in FIGS. 1 through 3; and
FIG. 6 is a detailed plan view of an alternate embodiment of an injection needle.

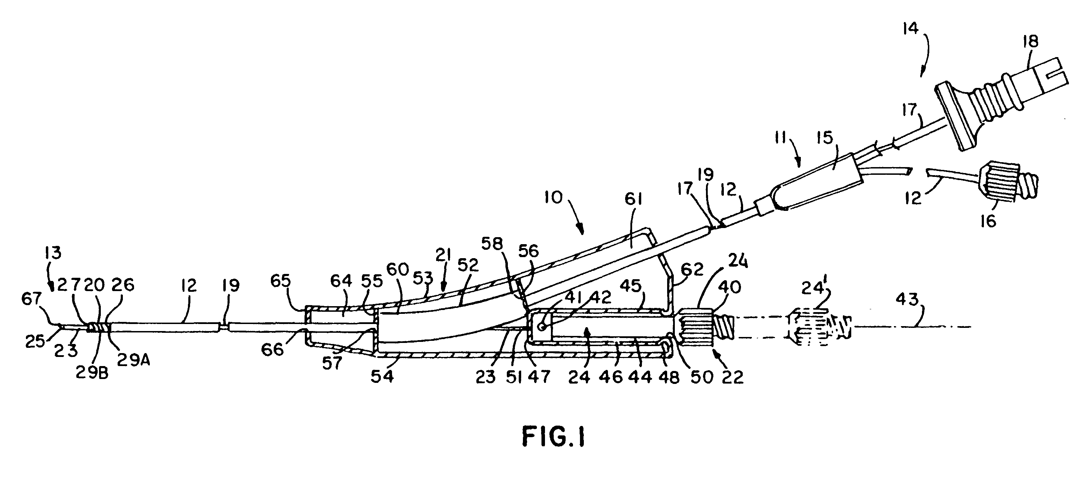

FIG. 1 discloses an integrated catheter assembly 10 that enables a physician to utilize diverse in situ therapy modalities at selected tissue sites without withdrawing the assembly 10 from the working channel or lumen of an endoscope. It includes a modified bipolar hemostat and irrigation system 11, such as the above identified Gold Probe hemostat. The system 11 enables a physician to utilize a dual purpose device for bipolar hemostasis and irrigation in the treatment of a bleeding vessel. The system 11 particularly includes a catheter 12 with a single lumen that extends from a distal location 13 to a proximal location 14. At the proximal location 14 a catheter hub 15 carries the catheter 12 from a Leur lock or similar catheter fitting 16 toward the distal location 13. Electrical leads 17 from an RF generator connector 18 also enter the catheter hub 15. RF generators of the type utilized with this apparatus are well known and therefore not shown. The electrical leads 17 are led into the center of the catheter 12 in the hub 15 thereby to be carried through a central lumen 19 of the catheter 12 to the distal location 13 and a bipolar electrode assembly 20. As an alternative, the catheter 12 may incorporate electrical leads in the catheter wall thereby to eliminate any contact between irrigating solutions in the catheter lumen 19 and the electrical leads 17. The bipolar electrode assembly 20, when energized over the electrical leads 17 that connect the bipolar electrode assembly 20 with the RF generator (not shown) through the RF generator connector 18, provides hemostatic therapy by heating tissue due to the inherent resistance of the tissue. Thus, the electrical leads and the RF generator connector 18 provide connection between the RF electrode assembly 20 and the RF generator (not shown) to enable the user to control the energization of the RF electrode assembly 20 and the heating of the tissue engendered thereby.

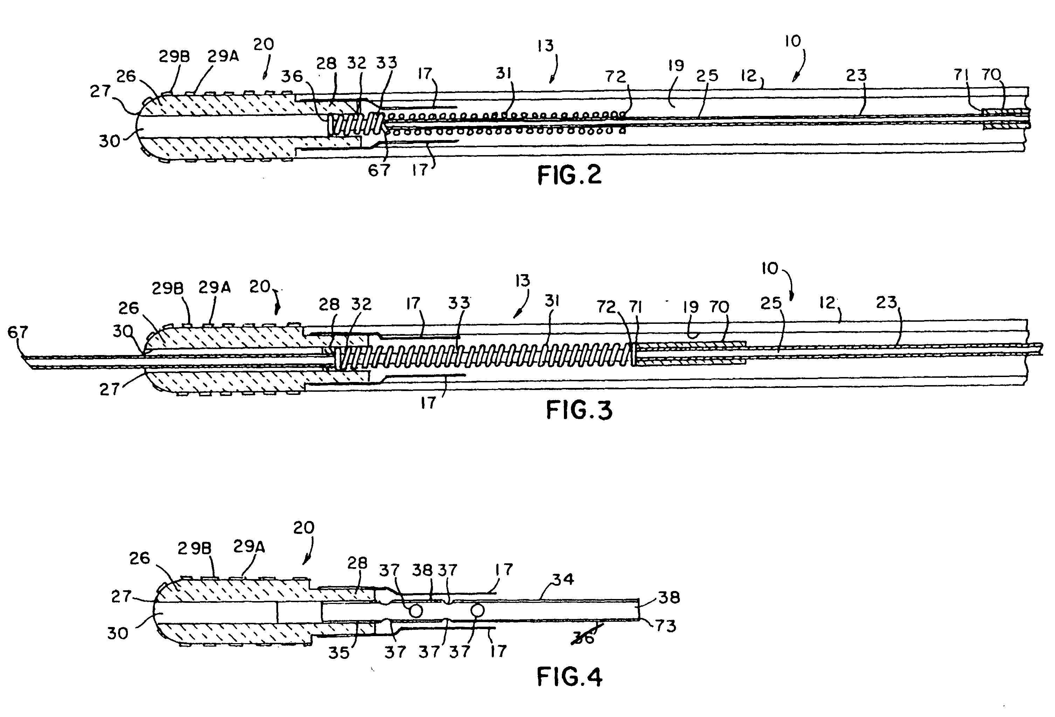

In accordance with this invention, a needle hub 21 directs the catheter 12 therethrough and supports the proximal end of a needle assembly 22 that includes an injection needle 23. The injection needle 23 can move between extended and retracted positions by manipulation of an operator 24. The operator 24 is shown at its extended position in FIG. 1 by the solid lines and in its retracted position by phantom operator 24'. When the needle 23 extends distally beyond the distal end of the bipolar electrode assembly 20 as shown in FIGS. 1 and 3, it can penetrate tissue and enable a physician to administer a vaso-constrictor or similar agent through a lumen 25 in the injection needle 23.

Referring now to different sections of the apparatus shown in FIG.1 in more detail, FIGS. 2 and 3 depict a distal end location 13 of the integrated catheter assembly 10. In each of FIGS. 2 and 3 the distal end of the catheter 12 terminates at the bipolar electrode assembly 20.

More specifically the bipolar electrode assembly 20 includes a cylindrical body portion 26 having a hemispherical distal end tip **27** and a proximally extending shank **28** at its other end. Discrete spiral electrodes **29A** and **29B** are disposed on the outer surface of the body portion **26** and the end tip **27** and connect to the electrical leads **17**. A distal tip/bipolar electrode assembly lumen **30** extends through the body portion end tip **27** and shank **28**. The shank **28** is nested and supported by the catheter **12**.

Still referring to **FIGS. 2** and **3**, a needle guide portion **31** includes an end section **32** that is located in the proximal end of the distal tip/bipolar electrode assembly lumen **30** and coextensive with a portion of the shank **28**. The needle guide portion **31** extends proximally from the shank **28** and constitutes a pervious guide tube for the needle **23**. More specifically, the needle guide **31** is formed as a spring with multiple spaced turns that define inter-turn passages **33.** These passages **33** allow fluid to transfer from the catheter lumen **19** and through the distal tip lumen **30** to exit from the end tip **27.** Fluid flow is relatively unimpeded in the structure shown in **FIG. 2** when the injection needle **23** is retracted. The extension of the needle **23** to the position shown in **FIG. 3** restricts the distal tip lumen **30,** but flow can still occur.

**FIG.4** depicts an alternative embodiment for the bipolar electrode assembly **20.** In this particular embodiment a tube **34** replaces the spring **31.** The tube **34** has a section **35** that fits in the distal tip lumen **30** and is coextensive with a portion of the shank **28** and another section **38** that is proximal of the shank **28.** This second section **38** includes a plurality of radially extending apertures **37** that act as passages for irrigation fluids from the catheter **12** through a central lumen **38.**

Thus **FIGS. 2** and **3**, and **FIG. 4,** respectively depict alternative embodiments of a bipolar electrode assembly **20** that includes first and second electrodes **29A** and **29B** for providing hemostatic therapy. In each embodiment a body portion **26** has a hemispherical distal end **27** and carriers the electrodes **29A** and **29B.** A shank **28** extends proximally of the body portion **26** for insertion of the catheter lumen **19** at the distal end of the catheter **12**. A tubular pervious needle guide **31** extends proximally from the shank portion **28** in the catheter lumen **19** to be coextensive with the distal portion of the catheter **12** for supporting the distal end of the injection needle **23** particularly in its retracted position.

Referring to **FIG. 1**, the operator **24** associated with the needle assembly **22** includes a proximal end fitting **40** that can connect to a syringe or other apparatus for enabling the injection of a vasoconstrictor or other therapeutic agent through the needle lumen **25**. At its opposite end, the operator **24** includes a collar **41** and set screw **42** or other attaching apparatus for affixing the operator **24** to the needle **23.** Such apparatus is known in the art. In this particular embodiment the operator **24** and needle **23** line along an axis **43.**

The needle hub **21** can be molded or otherwise formed to include a proximal first compartment **44** defined by side walls **45** and **46** and end walls **47** and **48.** An aperture **50** through the end wall **48** accommodates the operator **24** while an aperture **51** at the distal end wall **47** accommodates the needle **23.** The end walls **47** and **48** support the proximal end of the needle assembly **22** and limit the range of travel of the operator **24** along the axis **43** between the position shown in **FIG. 1** wherein the collar **41** abuts the wall **47** and a retracted position in which the collar **41** abuts the end wall **50.**

An intermediate second compartment **52** disposed distally of the proximal compartment **44** supports the catheter **12** in a radiused orientation. Curved and straight side walls **53** and **54** of the needle hub **21** and transverse end walls **55** and **56** define the compartment. The end wall **55** extends between the side wall **53** and **54**; the end wall **56**, between the side wall **53** and the intersection of the side wall **45** and end **47.** Apertures **57** and **58** in the end walls **55** and **56** respectively capture the catheter **12.**

An elastomeric seal **60** surrounds the catheter **12** and is located in the intermediate compartment **52.** The needle **23** penetrates the seal **60** and the wall of the catheter **12** thereby to be located in the catheter lumen **19** to extend through the distal tip lumen **30** as shown in **FIG. 2.** The seal **60** prevents leakage from the catheter **12** even during axial displacement of the needle **23** along the axis **43.** This seal **60** generally will be formed of an elastomeric material and can take any of several forms as known in the art.

The needle hub **21** includes another proximal compartment **61** adjacent the proximal compartment **44.** The compartment **61** is formed by a proximal end wall **62,** the side walls **45** and **52** and the end wall **56**. The end walls 56 and 62 in this compartment 61 support the catheter 12 proximally of the seal 60 and, with the compartment 52 and end wall 55, provides an angular offset to the catheter 12 with respect to the axis 43.

A further compartment comprising a distal compartment 64 is formed by the side walls 53 and 54, the end wall 55 and a distal end wall 65. An aperture 66 in the end wall 65 holds the catheter 12. The end walls 55 and 65 thereby maintain the alignment of the catheter 12 along the axis 43 to facilitate the placement and containment of the needle 23 within the catheter lumen 19 distally of the needle hub 21.

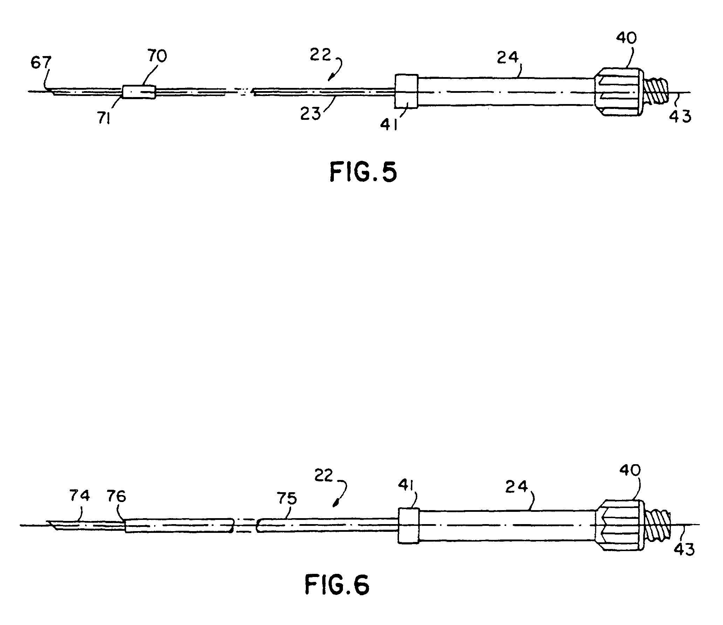

Still referring to FIG. 1, it is desirable to manufacture the needle hub 21 as a standard unit for a variety of applications. In some applications, the limits imposed on the axial travel of the injection needle 23 by the end walls 47 and 48 may allow an extension of the needle 23 from the bipolar electrode assembly 20 that is greater than desired. It is possible to customize that extension by applying a positive stop structure to the injection needle assembly 22. One such structure is shown in FIGS. 2, 3 and 5 where like numbers refer to like elements. As shown the needle assembly 22 particularly in FIG. 5 includes the operator 24 with its end fitting 40 and collar 41. The injection needle 23 extends as a constant diameter tube to its distal end 67. A collar 70 having a distal, radially extending end surface 71 is located on the needle 23 at some predetermined location spaced from the distal end 67 by a distance that equals the length of the desired extension plus the distance between the end tip surface 27 of the bipolar electrode assembly 20 as shown in FIG. 1 and a proximal end 72 of the needle guide 31 as shown in FIGS. 2 and 3. Consequently as the injection needle 23 moves from its retracted position in FIG. 2 to its extended position in FIG. 3, the distal end surface 71 of the collar 70, that overlies the spring 31, abuts the end 72 and prevents any further distal extension of the needle 23. If the bipolar electrode assembly 20 of FIG. 4 were used, the end surface 71 would abut an end surface 73 on the tube 34.

FIG. 6 discloses an alternative stop mechanism wherein the needle assembly 22 includes an operator 24 with proximal end connector 40 and distal collar 41. In this embodiment the needle assembly 22 comprises a distal hollow section 74 and a proximal hollow section 75. The distal section 74 has a given diameter corresponding to the diameter of the needle 23 shown in FIG. 5 and determined by the application requirements. The length of the distal section 74 equals the desired extension of the needle plus the distance from the distal end tip 27 to either end surface 72 of the spring 31 in FIGS. 2 and 3 or the end surface 73 of the tube 34 in FIG. 4. The proximal section 75 extends from the distal portion 74 to the operator 24 and has a larger diameter. Consequently the proximal portion 75 forms an annular radial surface 76 at its distal end that also will abut either the end 72 of the spring 31 in FIGS. 2 and 3 or the end 73 of the needle guide tube 34 shown in FIG. 4.

When a physician needs to treat a patient with internal bleeding, the physician will, as in the prior art, insert an endoscope with a working channel. Then the physician can insert the integrated catheter apparatus 10 shown in FIG. 1 through the working channel, normally with the injection needle 23 in its retracted position (as shown in FIG. 2). If it is necessary to irrigate the area, the physician can apply irrigating fluid through the connector 16 and the catheter lumen 19 to be ejected at the distal end tip 27 through the distal tip lumen 30 as shown in FIGS. 2 and 3. If upon viewing the site the physician decides to utilize hemostasis, it is merely necessary to position the bipolar electrode assembly 20 at the tissue and energize the electrodes 29A and 29B. The needle assembly 22 has no effect on this process. If, on the other hand, the physician determines the injection of a vaso-constrictor is appropriate before or in lieu of hemostasis, the physician can easily extend the injection needle 23 and administer a therapeutic agent through the connector 40 and the needle 23. Thereafter the physician can irrigate the site at will and elect to use hemostasis in addition to injection therapy. All these decisions are made and elections pursued without withdrawing the integrated catheter apparatus 10 from the endoscope. Moreover, each component requires the same basic manipulations as prior art devices familiar to a physician. Consequently time otherwise lost in manipulating individual elements in and out of the endoscope or in assuring proper operation of other combined apparatus is eliminated. This reduces the time for therapy and decreases the discomfort of the patient.

It will be helpful to describe some specific embodiments of this invention for the purpose of further understanding the construction and use of this invention. In certain specific embodiments, for example, the catheter assembly 10 can comprise a 21 mm (7 Fr.) or 30 mm (10 Fr.) catheter 20 and a 21 gauge needle 23. In an another embodiment, using a needle as shown in FIG. 6, the distal needle portion comprises a 23 - 25 gauge tubular structure while the proximal portion comprises a 21 to 22 gauge tubular structure. In addition, one embodiment of the catheter assembly 10 in FIG. 1 extends about 250 cm. between the distal tip portion 13 and the hub 21 while the extension of the needle 23 from the bipolar electrode assembly is limited to a maximum of 6 mm.

Although this invention has been described in terms of a specific embodiment, and certain modifications, still other modifications can be made. For example, needle assembly 22 can comprise a one-piece metal structure in the form shown in FIG. 5. In the form shown in FIG. 6 the distal portion might be constructed of a metal while the proximal portion 75 might be constructed of plastic. The needle assembly 22 also may include means for preventing rotation about the axis 43 during use.

## Claims

1. A catheter assembly **(10)** comprising:
catheter means **(12, 15, 16)** having a catheter lumen **(19)** therethrough from a proximal to a distal end thereof for extending from a location externally of a patient to tissue being treated, and
bipolar electrode means **(20)** attached to the distal end of said catheter means for providing hemostatic therapy to the selected tissue, said bipolar electrode means having a bipolar electrode means lumen **(30)** therethrough aligned with said catheter means lumen **(19)** for enabling the transfer of fluids between said catheter means lumen and the selected tissue through said bipolar electrode means lumen **(30)**, and
**characterised in that** the catheter assembly is an integrated assembly which additionally enables a physician to utilise dual in situ therapy modalities to treat internal bleeding at selected tissue, which dual modalities comprise a hemostatic therapy modality and an injection modality, and includes needle means **(22)** including an elongated hollow injection needle **(23)** having a needle lumen **(25)** therethrough for providing injection therapy, said needle being electrically isolated from said bipolar electrode means, having an axially directed distal portion, and extending from a proximal end externally of the patient through said catheter means lumen **(19)** and said bipolar electrode means lumen **(30)** for axial displacement whereby said distal portion of said needle **(23)** extends distally beyond and retracts proximally of the distal end surface **(27)** of said bipolar electrode means through said bipolar electrode means.

2. A catheter assembly as claimed in claim **1**, wherein:
i. said catheter means includes connecting means **(17, 18)** for controlling said electrode means, a catheter **(12)** with said catheter lumen **(19)** through which said needle **(23)** extends, and catheter hub means **(15)** at the proximal end of said catheter for carrying said catheter and for directing said connecting means through said catheter whereby said connecting means are carried to said electrode means, and
ii. said electrode means **(20)** includes first and second electrodes **(29A, 29B)**, a body portion **(26)** having said distal end surface **(27)**, for carrying said electrodes, a shank portion **(28)** extending proximally of said body portion for insertion in the catheter lumen **(19)** at the distal end of said catheter, and a cylindrical needle guide portion **(31, 34)** extending proximally from said shank portion to be coextensive with a distal portion of said catheter for supporting the distal end of said needle **(23)** in its retracted position.

3. A catheter assembly as claimed in claim **2**, wherein said needle guide portion **(31, 34)** is pervious thereby to enable the transfer of fluid between said catheter lumen **(19)** and said electrode means lumen **(30).**

4. A catheter assembly as claimed in claim **3**, wherein said needle guide portion **(31, 34)** comprises an elongated tube having one end portion **(32, 35)** inserted into said electrode means lumen **(30)** at the proximal end thereof and at least one radial aperture **(33, 37)** through said tube proximally of said electrode means for enabling the fluid transfer.

5. A catheter assembly as claimed in claim **4**, wherein said elongated tube is defined by an elongated spring **(32)** with axially spaced turns inserted into said electrode means lumen **(30)** at the proximal end thereof whereby the fluid transfer occurs through a radial aperture **(33)** defined by adjacent turns of said spring.

6. A catheter assembly as claimed in claim **2**, wherein said needle means **(22)** includes:
i. operator means **(24)** at the proximal end of said needle means for facilitating the transfer of fluid to said needle lumen **(25)**, and
ii: needle hub means **(21)** attached to said catheter intermediate said catheter hub means and said distal end of said catheter for guiding said elongated needle into said catheter lumen.

7. A catheter assembly as claimed in claim **6**, wherein said needle hub means includes a proximal compartment **(44)** for receiving and interacting with said operator means for displacement to enable the extension and retraction of said elongated needle, said proximal compartment including means **(40, 41)** for limiting proximal movement of said operator means

8. A catheter assembly as claimed in claim **7**, wherein said proximal compartment **(44)** comprises a first compartment, said needle hub means includes a second compartment **(52)** disposed distally of said proximal first compartment **(44)** for supporting said catheter in a radiused orientation, and sealing means **(60)** disposed in said second compartment **(52)** and around said catheter for enabling said needle to penetrate said sealing means and said catheter into said catheter lumen thereby to be movable between said extended and retracted positions.

9. A catheter assembly as claimed in claim **6**, wherein said needle hub means includes a proximal first compartment **(44)** for receiving said operator means, an intermediate second compartment **(52)** for supporting said catheter radiused orientation, a further distal compartment **(64)** for constraining a portion of said catheter, and sealing means **(60)** disposed in said intermediate second compartment and around said catheter for enabling said needle to penetrate said sealing means and said catheter into said catheter lumen thereby to be movable between said extended and retracted positions.

## Patentansprüche

1. Katheter-Baugruppe (10), **beinhaltend:**
Katheter-Mittel (12, 15, 16) mit einem Katheter-Lumen (19) hierdurch, von einem proximalen zu einem distalen Ende davon, um sich von einem Ort außerhalb eines Patienten zu einem zu behandelnden Gewebe zu erstrecken, und
ein Bipolar-Elektroden-Mittel (20), das an dem distalen Ende des Katheter-Mittels angeordnet ist, um dem ausgewählten Gewebe eine hämostatische Therapie zu verabreichen, wobei das Bipolar-Elektroden-Mittel ein Bipolar-Elektroden-Mittel-Lumen (30) hierdurch hat, das mit dem Katheter-Mittel-Lumen (19) fluchtet, um den Transport von Flüssigkeiten zwischen dem Katheter-Mittel-Lumen und dem ausgewählten Gewebe durch das Bipolar-Elektroden-Mittel-Lumen (30) zu ermöglichen, und
**dadurch gekennzeichnet, dass**
die Katheter-Baugruppe eine integrierte Baugruppe ist, welche einem Arzt zusätzlich ermöglicht, duale Ausführungsarten von Therapien in situ einzusetzen, um innere Blutungen bei ausgewählten Geweben zu behandeln, welche duale Ausführungsarten eine Ausführungsart einer hämostatischen Therapie und eine Ausführungsart einer Injektion beinhalten, und die Katheter-Baugruppe ein Nadel-Mittel (22) beinhaltet, das eine lang gestreckte hohle Injektions-Nadel (23) beinhaltet, mit einem Nadel-Lumen (25) hierdurch, um eine Injektionstherapie zu ermöglichen, wobei diese Nadel von dem Bipolar-Elektroden-Mittel elektrisch isoliert ist und ein axial ausgerichtetes distales Teil hat, und sich von einem proximalen Ende außerhalb des Patienten durch das Katheter-Mittel-Lumen (19) und das Bipolar-Elektroden-Mittel-Lumen (30) hindurch erstreckt, um axial verschoben werden zu können, wobei dieses distale Teil der Nadel (23) über die distale endseitige Oberfläche (27) des Bipolar-Elektroden-Mittel distal hinaus ausgefahren werden kann und hinter die distale endseitige Oberfläche (27) proximal zurückgezogen werden kann, durch das Bipolar-Elektroden-Mittel hindurch.

2. Katheter-Baugruppe nach Anspruch 1, **worin:**
i. das Katheter-Mittel ein Verbindungs-Mittel (17, 18) zum Regeln/Steuern des Elektroden-Mittels beinhaltet, einen Katheter (12) mit dem Katheter-Lumen (19), durch welches sich die Nadel (23) erstreckt, und Katheter-Vereinigungs-Mittel (15) an dem proximalen Ende des Katheters, zur Aufnahme des Katheters und zum Zuführen des Verbindungs-Mittels durch den Katheter, wobei die Verbindungs-Mittel zu dem Elektroden-Mittel geführt werden, und
ii. das Elektroden-Mittel (20) erste und zweite Elektroden (29A, 29B) beinhaltet, ein Körperteil (26) mit der distalen endseitigen Oberfläche (27) zur Aufnahme der Elektroden, ein Schaftteil (28), das sich proximal von diesem Körperteil erstreckt um in das Katheter-Lumen (19) an dem distalen Ende des Katheters eingeführt zu werden, und ein zylindrisches Nadel-Führungsteil (31, 34), das sich proximal von diesem Schaftteil erstreckt um sich gemeinsam mit einem distalen Teil des Katheters zu erstrecken, zur Aufnahme des distalen Endes der Nadel (23) in ihrer zurück gezogenen Position.

3. Katheter-Baugruppe nach Anspruch 2, **worin** das Nadel-Führungsteil (31, 34) durchlässig ist, um den Transport der Flüssigkeit zwischen dem Katheter-Lumen (19) und dem Elektroden-Mittel-Lumen (30) zu ermöglichen.

4. Katheter-Baugruppe nach Anspruch 3, **worin** das Nadel-Führungsteil (31, 34) ein lang gestrecktes Rohr beinhaltet, das ein Endteil (32, 35) hat, welches in das Elektroden-Mittel-Lumen (30) an dem proximalen Ende davon eingesetzt ist und mindestens eine radiale Öffnung (33, 37) durch das Rohr proximal zu dem Elektroden-Mittel hat, zur Ermöglichung des Transports der Flüssigkeit.

5. Katheter-Baugruppe nach Anspruch 4, **worin** das lang gestreckte Rohr durch eine lang gestreckte Feder (32) mit axial voneinander beabstandeten Windungen definiert ist, eingeführt in das Elektroden-Mittel-Lumen (30) an dem proximalen Ende davon, wobei der Transport der Flüssigkeit durch die radialen Öffnungen (33) geschieht, die definiert sind durch benachbarte Windungen dieser Feder.

6. Katheter-Baugruppe nach Anspruch 2, **worin** das Nadel-Mittel (22) beinhaltet:
i. Betätigungsmittel (24) an dem proximalen Ende des Nadel-Mittels, zur Erleichterung des Transports der Flüssigkeit zu dem Nadel-Lumen (25), und
ii. Nadel-Vereinigungs-Mittel (21), das an dem Katheter zwischen dem Katheter-Vereinigungs-Mittel und dem distalen Ende des Katheters angebracht ist, zum Einführen der lang gestreckten Nadel in das Katheter-Lumen.

7. Katheter-Baugruppe nach Anspruch 6, **worin** das Nadel-Vereinigungs-Mittel eine proximale Kammer (44) zur Aufnahme und Wechselwirkung mit dem Betätigungsmittel zum Verschieben beinhaltet, um das Aus- und Einfahren der lang gestreckten Nadel zu ermöglichen, wobei die proximale Kammer Mittel (40, 41) zur Begrenzung der proximalen Bewegung des Betätigungsmittels beinhaltet.

8. Katheter-Baugruppe nach Anspruch 7, **worin** die proximale Kammer (44) eine erste Kammer beinhaltet, wobei das Nadel-Vereinigungs-Mittel eine zweite Kammer (52) beinhaltet, die distal zur ersten proximalen Kammer (44) angeordnet ist zur Aufnahme des Katheters in einer radiusförmigen Richtung, und Abdicht-Mittel (60), das in der zweiten Kammer (52) und um den Katheter angeordnet ist, um es der Nadel zu ermöglichen, durch das Abdicht-Mittel und den Katheter in das Katheter-Lumen einzudringen, und dabei zwischen ausgefahrenen und zurück gezogenen Positionen bewegbar zu sein.

9. Katheter-Baugruppe nach Anspruch 6, **worin** das Nadel-Vereinigungs-Mittel eine erste proximale Kammer (44) zur Aufnahme des Betätigungsmittels beinhaltet, eine dazwischen liegende zweite Kammer (52) zur Aufnahme des Katheters in einer radiusförmigen Richtung, eine weitere distale Kammer (64) zur Begrenzung eines Teils des Katheters, und Abdicht-Mittel (60), das in der dazwischen liegenden zweiten Kammer und um den Katheter angeordnet ist, um es der Nadel zu ermöglichen, durch das Abdicht-Mittel und den Katheter in das Katheter-Lumen einzudringen, und dabei zwischen ausgefahrenen und zurück gezogenen Positionen bewegbar zu sein.

## Revendications

1. Ensemble de cathéter (10) comprenant :
des moyens de cathéter (12, 15, 16) comportant un lumen de cathéter (19) traversant à partir d'une extrémité proximale vers une extrémité distale de celui-ci pour s'étendre à partir d'une position située à l'extérieur d'un patient vers un tissu à traiter,
des moyens d'électrodes bipolaires (20) fixés à l'extrémité distale desdits moyens de cathéter pour fournir une thérapie hémostatique au tissu sélectionné, lesdits moyens d'électrodes bipolaires ayant un lumen de moyens d'électrodes bipolaires (30) traversant, aligné avec ledit lumen des moyens de cathéter (19) pour permettre le transfert de fluides entre ledit lumen de moyen de cathéter et le tissu sélectionné à travers ledit lumen de moyens d'électrodes bipolaires (30), et
**caractérisé en ce que** l'ensemble de cathéter est un ensemble intégré qui permet de manière additionnelle à un médecin d'utiliser des modalités de thérapie in situ duales pour traiter une hémorragie interne au niveau du tissu sélectionné, lesdites modalités duales comprenant une modalité de thérapie hémostatique et une modalité d'injection, et comprennent des moyens d'aiguilles (22) comportant une aiguille d'injection creuse allongée (23) ayant un lumen d'aiguille (25) à travers pour fournir une thérapie d'injection, ladite aiguille étant isolée électriquement desdits moyens d'électrodes bipolaires, ayant une partie distale dirigée axialement et s'étendant à partir d'une extrémité proximale à l'extérieur du patient à travers ledit lumen de moyens de cathéter (19) et ledit lumen de moyens d'électrodes bipolaires (30) pour un déplacement axial, de telle sorte que ladite partie distale de ladite aiguille (23) s'étend de manière distale au-delà et se rétracte de manière proximale de ladite surface d'extrémité distale (27) desdits moyens d'électrodes bipolaires à travers lesdits moyens d'électrodes bipolaires.

2. Ensemble de cathéter selon la revendication 1, dans lequel :
i. lesdits moyens de cathéter comprennent des moyens de connexion (17, 18) pour commander lesdits moyens d'électrodes, un cathéter (12) avec ledit lumen de cathéter (19) à travers lequel ladite aiguille (23) s'étend, et des moyens de douille de cathéter (15) à l'extrémité proximale dudit cathéter pour supporter ledit cathéter et pour diriger lesdits moyens de connexion à travers ledit cathéter, de telle sorte que lesdits moyens de connexion sont transportés auxdits moyens d'électrodes,
ii. lesdits moyens d'électrodes (20) comprennent une première et une seconde électrode (29a, 29b), une partie de corps (26) ayant ladite surface d'extrémité distale (27), pour supporter lesdites électrodes, une partie de tronc (28) s'étendant de manière proximale à partir de ladite partie de corps pour être insérée dans le lumen de cathéter (19) à l'extrémité distale dudit cathéter, une partie de guidage d'aiguille cylindrique (31, 34) s'étendant de manière proximale à partir de ladite partie de tronc pour être coextensive avec une partie distale du cathéter pour supporter l'extrémité distale de ladite aiguille (23) dans sa position rétractée.

3. Ensemble de cathéter selon la revendication 2, dans lequel ladite partie de guidage d'aiguille (31, 34) est perméable permettant ainsi le transfert de fluides entre ledit lumen de cathéter (19) et ledit lumen de moyens d'électrodes (30).

4. Ensemble de cathéter selon la revendication 3, dans lequel ladite partie de guidage d'aiguille (31, 34) comprend un tube allongé ayant une partie d'extrémité (32, 35) insérée dans ledit lumen de moyens d'électrodes (30) à l'extrémité proximale de celui-ci, et au moins une ouverture radiale (33, 37) à travers ledit tube de manière proximale auxdits moyens d'électrodes pour permettre le transfert de fluides.

5. Ensemble de cathéter selon la revendication 4, dans lequel ledit tube allongé est défini par un ressort allongé (32) avec des spires espacées axialement inséré dans ledit lumen de moyens d'électrodes (30) à son extrémité proximale, de telle sorte que le transfert de fluides se produit à travers une ouverture radiale (33) définie par des spires adjacentes dudit ressort.

6. Ensemble de cathéter selon la revendication 2, dans lequel lesdits moyens d'aiguilles (22) comprennent :
i. des moyens opérateurs (24) à l'extrémité proximale desdits moyens d'aiguille pour faciliter le transfert de fluides vers ledit lumen d'aiguille (25), et
ii. des moyens de douille d'aiguille (21) fixés audit cathéter entre lesdits moyens de douille de cathéter et ladite extrémité distale dudit cathéter pour guider ladite aiguille allongée dans ledit lumen de cathéter.

7. Ensemble de cathéter selon la revendication 6, dans lequel lesdits moyens de douille d'aiguille comprennent un compartiment proximal (44) pour recevoir et coopérer avec lesdits moyens opérateurs pour un déplacement pour permettre l'extension et la rétraction de ladite aiguille allongée, ledit compartiment proximal comprenant des moyens (40, 41) pour limiter un mouvement proximal desdits moyens opérateurs.

8. Ensemble de cathéter selon la revendication 7, dans lequel ledit compartiment proximal (44) comprend un premier compartiment, lesdits moyens de douille d'aiguille comprennent un second compartiment (52) disposé de manière distale à partir dudit premier compartiment proximal (44) pour supporter ledit cathéter dans une orientation courbée, et des moyens d'étanchéité (60) disposés dans ledit second compartiment (52) et autour dudit cathéter pour permettre à ladite aiguille de pénétrer lesdits moyens d'étanchéité et ledit cathéter dans ledit lumen de cathéter, pour être ainsi déplaçable entre lesdites positions étendue et rétractée.

9. Ensemble de cathéter selon la revendication 6, dans lequel lesdits moyens de douille d'aiguille comprennent un premier compartiment proximal (44) pour recevoir lesdits moyens opérateurs, un second compartiment intermédiaire (52) pour supporter ledit cathéter dans une orientation courbée, un compartiment distal supplémentaire (64) pour contraindre une partie dudit cathéter, et des moyens d'étanchéités (60) disposés dans ledit second compartiment intermédiaire et autour dudit cathéter pour permettre à ladite aiguille de pénétrer dans lesdits moyens d'étanchéité et audit cathéter de pénétrer dans ledit lumen de cathéter pour être ainsi déplaçable entre lesdites positions étendue et rétractée.
